# EUROPEAN PATENT APPLICATION

(11) **EP 3 701 906 A1**
(43) Date of publication of application: **02.09.2020**
(21) Application number: 18830295.4
(22) Date of filing: 26.10.2018
(51) Int. Cl.: A61C 8/02, A61C 8/00

(54) **DENTAL IMPLANT MADE OF ZIRCONIA OR ALUMINA WITH HEALING ELECTRICAL PROPERTIES AND ITS PRODUCTION METHOD**

(30) Priority: 26.10.2017 PT 2017110375
(71) Applicant: Universidade do Minho, 4704-553 Braga (PT)
(72) Inventor: CORREIA PEREIRA SILVA, Filipe Samuel, 4800-058 Guimarães (PT); SALGADO PINTO, Paulo Filipe, 4800-058 Guimarães (PT); NOVAIS CARVALHO, Óscar Samuel, 4800-058 Guimarães (PT)
(74) Representative: Patentree
(86) International application number: PCT/IB2018/058397
(87) International publication number: WO 2019/082154

(57) **Abstract**

The present disclosure relates to a dental implant which comprises in its interior vertical and radial channels terminating in the form of holes in the lateral and lower surfaces of the implant and wherein each channel is filled by electrically conductive material in order to promote multiple electric fields along the surface of the implant. Electric fields range from 5 to 100 mv. A dental implant is described defining an axis along its length, obtained from a sintered block, which comprises in its interior longitudinal and radial channels which terminate in the form of holes in the surface of the implant and wherein each channel comprises an electrically conductive material for promoting multiple electric fields along the surface of the implant.

## Description

### Technical domain

The present disclosure is in the area of dentistry, more specifically in the area of dental implants. The present disclosure relates to a ceramic dental implant with electrical capacity for bone regeneration and anti-bacterial effect.

### Background

There are several proposals for dental implants that have electrical stimuli with the purpose of aiding the regeneration of bone tissues adjacent to the implant. Most of the proposals, including the following examples of patents US8,374,697, CN103006343 and WO2006043748, propose an implant where the electrodes are mounted on the top and bottom of the implant, respectively, both being mounted on the main body of the implant. The electrode that acts on the lower part of the implant passes through the inside of the implant. The implant described in these documents allows the creation of an electric field (and magnetic) between these two poles.

Another approach, set forth in patent document US2003 / 0153965, proposes the use of composite materials of polymer matrix and / or ceramic for being used, together with electrically conductive reinforcements, for example carbon nanotubes, to transmit electric current along two poles of the implant. In turn, the patent document US5738521 proposes the placement of the two electrical poles, one in the implant and the other in an area of the bone, outside the implant, in order to create an electric field between these two points.

In all the solutions presented there are always two poles, mounted on distinct points of the implant, usually at the end points of the implant, or even one of the poles is placed outside the implant, between which an electric field is created. This electric field will generate electric currents between the two poles and thus stimulates bone growth in the zones of current flow through the tissues of the mandible and / or maxilla. The electric current will flow randomly through the areas of lower electrical resistance of bone tissues.

A new concept of implant is proposed in this document and consists in a device where multiple electric fields are created, allocated at multiple points of the implant surface strategically defined, the electrical wire circuit being an integral part of the component, also contributing to its mechanical strength. The way of obtaining the implant is also proposed in this document.

These facts are described in order to illustrate the technical problem addressed by this disclosure.

### General Description

The present disclosure relates to a ceramic dental implant based on zirconia or alumina, or mixtures thereof, which internally comprises an electric circuit that allows electric fields to reach multiple points of the surface of the implant, allowing this electric circuit to form an integral part of the body of the implant.

As stated, the implant is composed of a ceramic or ceramic composite, biocompatible, with an aesthetic function, which may comprise bioactive and / or antibacterial compounds. This implant is characterized by comprising in its interior an electric circuit intended for providing electric fields to multiple points of the implant surface, through positively and negatively polarized electrical wires reaching multiple points of the surface of the implant. For this purpose, the implant comprises in its interior vertical distribution channels along the main direction of the implant, and radial ones at various implant heights, which will allow multiple electric fields to exist on the implant surface. With the electrical currents transmitted by the implant to the bone, will allow its regeneration on a quicker way, as well as will have an anti-bacterial effect, helping to eliminate biofilms of bacteria. This circuit is powered by a small battery that housed at the top of the implant, or inside the abutment or crown, together with a chip that coordinates the electrical stimuli, namely electric field intensity, frequency of application, and duration of application, and / or alternatively by a piezoelectric component, which is actuated by the mastication force. The piezoelectric component shall consist of a biocompatible material, for example Barium Titanate BaTiO3, or 'niobate perovskite' compounds, ((K, Na) NbO3), called KNN, and generates an electric field when subjected to a deformation due to a load such as that of mastication.

The ceramic should be based on zirconia or alumina, or mixtures thereof, possibly containing hydroxyapatite, βTCP, bioglass, among other materials that stabilize the ceramic or zirconia, and / or bioactive and / or anti-bacterial.

The material of the electric circuit consists of silver or gold or platinum or other biocompatible metal or other biocompatible and electrically conductive material.

The electric circuit is characterized as being an integral part of the implant not being an assembly of wires or electrodes. The electrical circuit is responsible for emission of the electric fields to the various points of the implant but it also contributes for the mechanical strength of the same.

The process for obtaining the implant is based on the following approach. Compaction and pre-sintering of a block from zirconia and / or alumina powders, which may contain zirconia stabilizing elements such as yttria, ceria, Magnesia, among others, and may also contain bioactive elements such as hydroxyapatite, βTCP, bioglass, or other bioactive materials. Alternatively already compacted and pre-sintered commercial blocks may be used; followed by machining of the pre-sintered block by mechanical route with cutting tools or by laser ablation, for the creation of the internal channels, electric field distributors to the implant surface (lateral and inferior); followed by final sintering of the component; followed by filling of the channels of the zirconia block with an electrically conductive and bio compatible material such as silver, gold, platinum, or a polymer such as Polyether ether ketone (PEEK), Poly (methyl methacrylate) (PMMA), or other biocompatible polymer, charged with micro or nano metal particles or other reinforcement that renders the polymer electrically conductive, using a casting process under pressure; followed by assembly of the control unit, the battery, and / or the piezoelectric component, on the top of the implant, on the abutment, or on the inside of the crown.

The present proposal presents several innovations and advantages, namely:

The first major advantage of the implant proposed in this document is the existence of multiple electric fields created on the surface of the implant, due to the existence of multiple negative and positive electrodes. In this way, instead of a single electric field multiple electric fields are created dispersed on the surface of the implant, thus making the stimulation for bone regeneration and antibacterial effect much more efficient. Instead of a single electric field created around the implant, which will cause a randomly current flow through the tissues, thus being effective where the current flows, but less effective where current does not flow, this proposal presents a solution for the creation of current flows along the entire surface of the implant, making the process of bone regeneration and antibacterial effect, through electrical stimulation, much more effective, ensuring its effect on the entire surface of the implant.

The second major advantage of the proposed implant is that the wiring circuit within the implant is an integral part of the implant body thus contributing for its mechanical strength. In this proposal, and contrary to the existing solutions where the electrical poles are mounted on the implant, thus constituting additional components of the implants, in this proposal the electric circuit is integrated into the implant, forming a composite material.

The third major advantage of the proposed implant is that it can work operated by a battery or by mastication forces. In an initial phase, where, intentionally there may be no mastication loads, the electrical stimuli will be promoted by a control unit, which will control the frequency, timing, and power of electric fields, and by a battery, which will supply the electric power. In a second stage, where mastication forces already exist, this control unit and battery are replaced by a piezoelectric device that will create the electric fields when actuated by mastication forces. This advantage allows that, contrarily to existing patents, this solution operates during the secondary stabilization period, i.e. in the first months after placement of the implant, using the battery, as well as throughout the whole life of the implant, using the piezoelectric element. This solution allows to mimic the piezoelectric effect of the collagen contained in the bone making this implants solution closer to the natural functioning of the bone itself.

A dental implant is described defining an axis along its length, obtained from a sintered block, which comprises in its interior longitudinal and radial channels terminating in the form of holes in the surface of the implant and wherein each channel comprises an electrically conductive material to create multiple electric fields along the surface of the implant. By throughout the implant is meant along said longitudinal axis of the implant.

In one embodiment, the radial channels terminate in the form of holes in a lateral surface of the implant and the longitudinal channels terminate in the form of holes on a top surface of said implant.

In one embodiment, the radial channels terminate in the form of holes in a lateral surface, the respective electrically conductive material being connected in such a way as to alternate electrical poles along the surface of the implant.

In one embodiment, the longitudinal and radial channels terminate in the form of holes in the lower lateral surface of the implant.

An embodiment comprises an electronic component located on the abutment or crown which comprises a battery having an integrated circuit, said chip, or a piezoelectric component.

In one embodiment, the electronic component is configured so that said electric fields range from 5 to 100 mv.

In one embodiment, the electrically conductive material is metal or polymer charged with particles or fibers that render the polymer electrically conductive.

In one embodiment, the metal is selected from silver or gold or platinum or other electrically conductive and biocompatible metal or metal alloy.

In one embodiment, the polymer is selected from PEEK or PMMA or other biocompatible polymer.

In one embodiment, the polymer further comprises particles of silver or carbon nanotubes, or other biocompatible material that renders the polymer electrically conductive.

In one embodiment, the sintered implant block is of ceramic material or ceramic composite.

In one embodiment, said sintered implant block is of zirconia or alumina or a mixture thereof.

In one embodiment, said implant comprises from 1 to 20% yttria and / or ceria and / or Magnesia.

An embodiment comprises hydroxyapatite and / or βTCP and / or bioglass.

A process for obtaining the dental implant of any one of the described embodiments is also described comprising the following steps:
mixing the constituent ceramic materials of the implant and press them with a pressure between 10 and 200 MPa until a compact block is obtained;
pre-sintering the obtained block;
machining the blocks by milling or by laser ablation in order to obtain the implants with the longitudinal and radial channels and the respective holes;
sintering the blocks at a temperature between 1200 and 1600 ° C for a period of 1 to 5 hours.

In one embodiment, the lost wax casting process comprises the following steps:
placing the implants in the feeding system, in the form of a tree, composed by wax;
adding wax to the implant holes;
immersing the implant-containing feeding system in plaster until a plaster mould is obtained;
heating the mould to a temperature of 400 ° C for extraction of the wax;
placing the mould in a furnace at 800 ° C for a period of 2 hours;
removing the mould and pouring the metal into the plaster mould and trough the holes of the implant.

In one embodiment, the filling of the inner channels by an electrically conductive polymer comprises the following steps:
the implant with the holes is placed inside a mould of graphite material or similar in which the mould is comprised of a main body, a lower part, and an upper part, together with the implant;
the polymer is placed inside the mould and these are heated until the polymeric composite reaches the liquid, pasty or semi-solid state, and impregnates the holes.

In one embodiment, the impregnation of the holes is carried out by applied pressure, under vacuum, which causes the polymeric composite to flow into the holes of the implants to fill them.

### Brief Description of the Drawings

For an easier understanding, the figures are attached, which represent preferred embodiments which are not intended to limit the object of the present description.

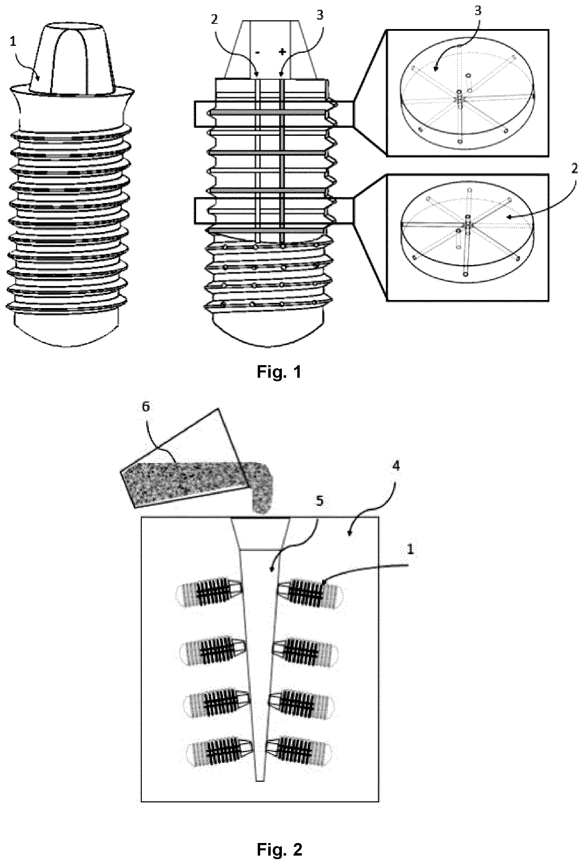

**Figure 1** - exemplary schematic representation of a ceramic implant constituted by the main body (1) (a), before machining the inner channels, which will give rise to the internal electric circuit, and after machining the internal channels (b), which will give rise to the internal electric circuit, and local for housing the battery and electronic components or piezoelectric element on the top of the implant. Also shown are cuts with top views (c) of the electric wire system showing the circuit with a polarity, for example the negative (2) and with the other polarity, for example the positive (3).
**Figure 2** - exemplary schematic representation of a mould in plaster (4), containing the implants (1), and on which metal will be poured in the liquid state (6), which will flow through the feeding channel (5), and which will fill the electric circuit (2) and (3) in Fig. 1) of the implants (1).

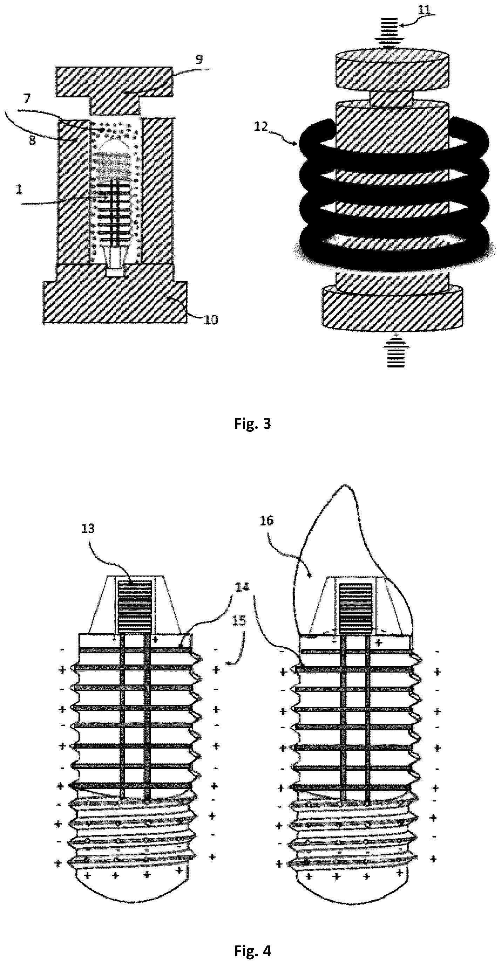

**Figure 3** - exemplary schematic representation of a mould, for example in graphite, constituted by a main body (8), and for example a lower part (10) and an upper part (9), where the implant (1) is placed with the channels which will constitute the electrical circuit and the location for the electronic component and battery, or piezoelectric component, already made, and where the powder or granules of the polymeric material, which is electrically conductive (7) are also placed. Pressure (11) and temperature (12), preferably in vacuum, is applied to fill the channels which will constitute the electric circuit by the polymeric composite.
**Figure 4** - exemplary schematic representation of a ceramic implant constituted by the main body, after machining and filling (14) of the internal channels that constitute the internal electric circuit, and example of electric fields (15) created, and battery and electronic components or piezoelectric element (13), and on which the crown (16) will be mounted.

### Detailed Description

The present disclosure consists in a dental implant, which is made up of a ceramic or ceramic composite (1) (Figure 1), biocompatible, with an aesthetic function, and possibly bioactive, containing in its interior an electric circuit (2) (3) intended for providing electric fields to multiple points of the implant surface (15). This circuit is powered by a small battery that is housed on the top of the implant, in a possible abutment, or in the crown, together with a chip that coordinates the electrical stimuli (13), and / or alternatively by a piezoelectric component (13), which is actuated by the mastication force.

The present disclosure consists in a dental implant, which is made up of a ceramic or ceramic composite (1) (Figure 1), biocompatible, with an aesthetic function, and possibly bioactive, containing in its interior an electric circuit (2) (3) which is intended for providing electric fields to multiple points of the implant surface (15). This circuit is powered by a small battery that is housed on the top of the implant, in a possible abutment, or in the crown, together with a chip that coordinates the electrical stimuli (13), and / or alternatively by a piezoelectric component (13), which is actuated by the mastication force.

In a preferred mode, the implant material consists of a ceramic based on zirconia, possibly containing alumina percentages or on alumina possibly containing zirconia. In addition to those, it may contain small percentages, for example from 1% (v / v) to 20% (v / v) of yttria, ceria, Magnesia, among other zirconia stabilizing materials, or even hydroxyapatite, βTCP, bioglass, among other bioactive and / or anti-bacterial materials.

These materials, after being mixed, are pressed with pressures from 10 to 200MPa, preferably between 50 and 100 MPa. The blocks may then be pre-sintered at temperatures between 800 ° C and 1200 ° C, for periods between 0.5 and 5 hours. Alternatively, commercially available pre-sintered blocks, may be used.

The pressed and / or pre-sintered blocks are then subjected to machining by mechanical route, with cutting tools, in numerically controlled equipments, for the creation of an housing area of the electronic and piezoelectronic components, and internal channels (2) (3), which will consist in the electric circuit. These channels will have a vertical distribution, along the main direction of the implant, and a radial one, at various implant heights. Alternatively, the laser ablation method for machining the holes that will consist in the internal channels may be used. The positive and negative electrical poles are in pairs (+ -) along the implant surface, in order to create multiple micro electric fields (15).

The machined blocks will be followed by final sintering of the component, which will occur at temperatures between 1200 ° C and 1600 ° C, for periods between 1h and 5h, between periods of heating and cooling, which can last between 2 to 4 hours each with heating ramps of about 5 ° C / minute.

After final sintering the implants will be subjected to filling of their internal channels with an electrically conductive material. In case the conductive material is metallic, for example silver or gold or platinum, among others that are biocompatible, these will be impregnated in the ceramic implant using the lost wax casting process. Briefly, the implants will be mounted on a feeding system (5), for example in the shape of a tree, in wax, the holes being filled with wax. Then the wax tree, which contains the implants, is immersed in plaster, inside a container giving rise to a plaster mould (4). Thereafter, this mould is subjected to a temperature of up to about 400 ° C for around 30 minutes, for extraction of the wax. Then the plaster mould is subjected to a baking cycle of the plaster in which it reaches about 800 ° C for a period of about 2 hours. Finally, the plaster mould is removed from the furnace and the liquid metal (6) which will consist in the electric circuit is poured into the interior of the mould (5), which contains the implants, filling the mould cavities and the internal holes of the implants (2) and (3).

In the case where the conductive material is of polymer matrix, for example Polyether ether ketone (PEEK), or Poly (methyl methacrylate (PMMA), or other biocompatible polymer charged with a material that renders it electrically conductive, for example silver particles, carbon nanotubes, among others, this polymer matrix composite (7) is placed inside a mould, for example in graphite, for example constituted by a main body (8), a lower part (10), and an upper part (9), together with the implant (1) already containing the holes (2) and (3), and these are heated (12) until the polymeric composite reaches the liquid, pasty or semi-solid state and impregnates the holes. This impregnation is done due to the applied pressure (11), under vacuum, which causes the polymeric composite to flow into the implant holes, filling them completely.

The implants will now have a geometry close to the final one, with the electric channels completely filled (14). They may possibly be subjected to machining for a final definition of geometry or thread. They may also be subjected to a polishing, or surface treatment by particle blasting, acid etching, laser, among others intended to create a surface texture suitable for a good bond to the bone tissues.

At the top of the implant, or at the abutment, if the implant has it, it can then be mounted the signal processing station and the battery (13), or alternatively the piezoelectric component (13), which will give rise to the electric fields, distributed along the surface of the implant (15). The electric fields on the surface of the implant may be in the range of 5 to 100 mv. The crown (16) will be cemented or screwed over the implant body. In the event that the electronic system is made up of a controller and a battery, these (13) must operate while there are no mastication forces, and can then be removed and the electric fields become promoted by a piezoelectric component, which will actuate with the forces of mastication, throughout the entire life of the implant. The piezoelectric component consists of a biocompatible material, for example Barium Titanate BaTiO3, or niobate perovskite compounds, ((K, Na)NbO3), called KNN, and which generate an electric field when subjected to a deformation, due to a load such as that of mastication.

### Bibliographic Refs.

[1] US 8,374,697 B2 - Electrical dental screw implant
[2] CN 103006343 B - Dental implant micro-electrical stimulation healing device
[3] WO 2006043748 A1 - Apparatus for accelerating osseointergration
[4] US 2003/0153965 A1 - Electrically conducting nanocomposite materials for biomedical applications.
[5] US 5738521 A - Method for accelerating osseointegration of metal bone implants using electrical stimulation.

In one embodiment of the present disclosure the dental implant can comprise in its interior vertical channels (i.e., longitudinal channels along the dental implant defining an axis along its length) and radial ones that terminate in the form of holes in the lateral and lower surface of the implant and in which each channel comprises an electrically conductive material so as to promote multiple electric fields along the surface of the implant.

In one embodiment, the implant may comprise an electronic component located on the abutment or crown consisting in a battery with a chip or in a piezoelectric component.

In one embodiment, the electric fields range from 5 to 100 mv.

In one embodiment, the electrically conductive material may be metal or polymer charged with particles or fibers that render the polymer electrically conductive.

In one embodiment, the metal is selected from silver or gold or platinum or another biocompatible and electrically conductive metal.

In one embodiment, the polymer may be selected from PEEK or PMMA or another biocompatible polymer.

In one embodiment, the polymer may further comprise particles of silver or carbon nanotubes, or other biocompatible material that renders the polymer electrically conductive.

In one embodiment, the implant may be of ceramic or ceramic composite material.

In one embodiment, the implant may be of zirconia or alumina or a mixture thereof.

In one embodiment, the implant may further comprise from 1 to 20% of yttria and / or ceria and / or Magnesia.

In one embodiment, the implant may further comprise hydroxyapatite and / or TCP and / or bioglass.

In one embodiment, the process for obtaining the implant may comprise the following steps:
a. mixing the constituent ceramic materials of the implants and press them at a pressure between 10 and 200 MPa until a compact block is obtained;
b. pre-sintering the obtained block;
c. machining the blocks by milling in order to obtain the implants with the vertical and radial channels and the respective holes;
d. sintering the blocks at a temperature between 1200 and 1600 ° C for a period of 1 to 5 hours.

In one embodiment, the process may comprise channels which may be produced alternatively by laser ablation.

In one embodiment, the lost wax casting process may comprise the following steps:
a. placing the implants in the feeding system, tree-shaped, composed of wax;
b. adding wax to the implant holes;
c. immersing the implant-containing feeding system in plaster until a plaster mould is obtained;
d. heating the mould to a temperature of 400 ° C for extraction of the wax;
e. placing the mould in a furnace at 800 ° C for a period of 2 hours;
f. removing the mould and pouring the metal into the plaster mould and into the holes of the implant.

In one embodiment, the process of filling the inner channels with an electrically conductive polymer may comprise the following steps:
a. The implant with the holes is placed inside a mould of graphite material or similar, wherein the mould is constituted by a main body, a lower part, and an upper part, together with the implant;
b. The polymer is placed inside the mould and these are heated until the polymeric composite reaches the liquid, pasty or semi-solid state, and impregnates the holes.

In one embodiment, the impregnation process of the holes may be made by pressure applied under vacuum, which causes the polymeric composite to flow into the orifices of the implants, filling them completely.

The present disclosure is not, of course, in any way restricted to the embodiments described in this document and a person with average knowledge in the art may provide many possibilities of modifying it and replacing of technical characteristics by equivalent ones, depending on the requirements of each situation, as defined in the appended claims.

The following claims further define preferred embodiments.

## Claims

1. Dental implant defining an axis along its length, obtained from a sintered block, which comprises in its interior longitudinal and radial channels terminating in the form of holes in the surface of the implant and wherein each channel comprises an electrically conductive material for creating multiple electric fields along the surface of the implant.

2. Dental implant according to the preceding claim, wherein the radial channels terminate in the form of holes in a lateral surface of the implant and the longitudinal channels terminate in the form of holes in a top surface of said implant.

3. Dental implant according to any one of the preceding claims, wherein the radial channels terminate in the form of holes in a lateral surface, the respective electrically conductive material being connected in such a way as to alternate electrical poles along the surface of the implant.

4. Dental implant according to any one of the preceding claims, wherein the longitudinal and radial channels terminate in the form of holes in the lateral and lower surfaces of the implant.

5. Dental implant according to any one of the preceding claims, comprising an electronic component located on the abutment or crown, which comprises a battery having an integrated circuit, said chip, or a piezoelectric component.

6. Dental implant according to the preceding claim, wherein the electronic component is configured so that said electric fields range between 5 and 100 mv.

7. Dental implant according to any one of the preceding claims, wherein the electrically conductive material is metal or polymer charged with particles or fibers that render the polymer electrically conductive.

8. Dental implant according to the preceding claim, wherein the metal is selected from silver or gold or platinum or other metal or metal alloy biocompatible and electrically conductive.

9. Dental implant according to claim 7, wherein the polymer is selected from PEEK or PMMA or other biocompatible polymer.

10. Dental implant according to the preceding claim, wherein the polymer further comprises silver particles or carbon nanotubes, or other biocompatible material that renders the polymer electrically conductive.

11. Dental implant according to any one of the preceding claims, wherein the sintered implant block is of ceramic or ceramic based composite material.

12. Dental implant according to the preceding claim, wherein said sintered implant block is of zirconia or alumina or a mixture thereof.

13. Dental implant according to the preceding claim, wherein said implant comprises from 1 to 20% of yttria and / or ceria and / or Magnesia.

14. Dental implant according to any one of the preceding claims, comprising hydroxyapatite and / or βTCP and / or bioglass.

15. Process for obtaining the dental implant of any one of claims 1 to 14 comprising the following steps:
mixing the constituent ceramic materials of the implant and press them at a pressure between 10 and 200 MPa until a compact block is obtained;
pre-sintering the obtained block;
machining the blocks by milling or by laser ablation in order to obtain the implants with the longitudinal and radial channels and the respective holes;
sintering the blocks at a temperature between 1200 and 1600 ° C for a period of 1 to 5 hours.

16. Process according to the preceding claim, wherein the lost wax casting process comprises the following steps:
placing the implants in the feeding system, in the form of a tree, composed by wax;
adding wax to the implant holes;
immersing the implant-containing feeding system in plaster until a plaster mould in is obtained;
heating the mould to a temperature of 400 ° C for extraction of the wax;
placing the mould in a furnace at 800 ° C for a period of 2 hours;
removing the mould and pouring the metal into the plaster mould and into the holes of the implant.

17. Process according to any one of claims 15-16, wherein the filling of the inner channels by an electrically conductive polymer comprises the following steps:
the implant with the holes is placed inside a mould of graphite material or similar in which the mould is comprised by a main body, a lower part, and an upper part, together with the implant;
the polymer is placed inside the mould and these are heated until the polymeric composite reaches the liquid, pasty or semi-solid state, and impregnates the holes.

18. Process according to anyone of claims 15-17, wherein the impregnation of the holes is done by applying pressure under vacuum, which causes the flow of the polymeric composite into the holes of the implants, in order to fill them.
